# EUROPÄISCHE PATENTANMELDUNG

(11) **EP 0 893 098 A2**
(43) Veröffentlichungstag der Anmeldung: **27.01.1999**
(21) Anmeldenummer: 98112628.7
(22) Anmeldetag: 08.07.1998
(51) Int. Cl.: A61B 17/17, A61B 19/00

(54) **Führungsvorrichtung für Instrumente, insbesondere für chirurgische Instrumente**

(30) Priorität: 21.07.1997 DE 19731194
(71) Anmelder: Sandhaus, Sami, Dr., CH-1007 Lausanne (CH)
(72) Erfinder: Sandhaus, Sami, Dr., CH-1007 Lausanne (CH)
(74) Vertreter: Hiebsch, Gerhard F., Dipl.-Ing.

(57) **Zusammenfassung**

Bei einer Führungsvorrichtung für Instrumente -- insbesondere für chirurgische Instrumente -- zum Herstellen von Kavernen wie Vertiefungen und Bohrungen für Implantate in Knochengeweben ist ein das Instrument aufnehmender Instrumentenhalter an der an einem Festpunkt festlegbaren Führungsvorrichtung in wenigstens zwei einander querenden Richtung verlagerbar sowie um zumindest eine Gelenkachse drehbar angeordnet. Der bevorzugt in drei Koordinatenrichtungen verschiebbar gelagerte Instrumentenhalter soll zudem um die Gelenkachse in einem Winkel von mehr als 90° --bevorzugt 360° -- drehbar sowie um eine diese Gelenkachse querende zweite Gelenkachse schwenkbar angeordnet sein.

## Beschreibung

Die Erfindung betrifft eine Führungsvorrichtung für Instrumente, insbesondere für chirurgische Instrumente zum Herstellen von Kavernen -- wie Vertiefungen und Bohrungen -- für Implantate in Knochengeweben.

Zum Einsetzen von Implantaten in der Chirurgie, der Orthopädie oder Kieferchirurgie sind durch Bohren oder Fräsen herzustellende Bohrungen und Vertiefungen -- vor allem Langlöcher -- erforderlich. In den meisten Fällen werden diese dadurch hergestellt, daß auf die mit der Kaverne zu versehende Stelle eine Bohrlehre aufgesetzt wird, die als Führung das Bohren mit einer relativ hohen Genauigkeit erlaubt. Die Erzeugung eines Langloches ist wesentlich schwieriger, da die Querbewegung des Bohrers oder Fräsers von Hand durchzuführen ist, somit eine erhebliche Fertigkeit des Operierenden voraussetzt. Zum Einhalten der genauen Tiefe dieser Langlöcher setzt man Anschläge ein, die an der Bohrmaschine bzw. am Bohrkopf angebracht werden.

Bei allen Arbeiten, bei denen Bohrungen bzw. Langlöcher im Knochengewebe operativ hergestellt werden müssen, bleibt der Einfluß der Fertigkeit des Operierenden eine risikoreiche Begleiterscheinung auch dann, wenn die erwähnten, in den meisten Fällen nach Röntgenbildern hergestellten Bohrlehren die Genauigkeit verbessern helfen.

Bei allen bekannten Vorrichtungen und Lehren im Bereich der Operationstechnik fehlt die Möglichkeit, Bohrungen oder Langlöcher mit hoher Präzision halbautomatisch oder automatisch herzustellen. Nicht zuletzt deshalb müssen sehr lange Operationszeiten vorgesehen und hohe Kosten hingenommen werden.

In Kenntnis dieser Gegebenheiten hat sich der Erfinder das Ziel gesetzt, eine Führungsvorrichtung der eingangs erwähnten Art zu schaffen, welche eine genaue Herstellung der Kavernen -- insbesondere von Langlöchern -- erlaubt, die Operationszeiten verkürzt und die Kosten mindert.

Zur Lösung dieser Aufgabe führt die Lehre des unabhängigen Patentanspruchs; die Unteransprüche geben günstige Weiterbildungen an. Zudem fallen in den Rahmen der Erfindung alle Kombinationen aus zumindest zwei der in der Beschreibung, der Zeichnung und/oder den Ansprüchen offenbarten Merkmale.

Erfindungsgemäß ist ein das Instrument aufnehmender Instrumentenhalter an der -- an einem Festpunkt festlegbaren --Führungsvorrichtung in wenigstens zwei einander querenden Richtung verlagerbar sowie um zumindest eine Gelenkachse drehbar angeordnet. Dabei hat es sich als günstig erwiesen, wenn der Instrumentenhalter in drei Koordinatenrichtungen verschiebbar und/oder um die Gelenkachse in einem Winkel von mehr als 90° -- bevorzugt 360° -- drehbar gelagert ist. Besonders vorteilhaft ist es, den Instrumentenhalter auch um eine die Gelenkachse querende zweite Gelenkachse schwenkbar anzuordnen.

Die erfindungsgemäßen Maßgaben schaffen also eine Führungsvorrichtung mit einem Instrumentenhalter für den Bohr- und Fräskopf, der in mehr als zwei Achsen lineargenau und zudem mit hoher Präzision drehbar sowie neigbar einzustellen ist.

Nach einem weiteren Merkmal der Erfindung ist der an einem Arbeitskopf der Führungsvorrichtung gelagerte Instrumentenhalter quer zu deren Längsachse an einem Schlitten verschiebbar sowie mit diesem anhebbar ausgestaltet. Er sitzt bevorzugt seitlich des Arbeitskopfes an einer diesen überragenden Lasche od.dgl. Materialstreifen.

Für die die Längsachse querende Bewegungsbahn des Instrumentenhalters sowie die Hubbahn des Arbeitskopfes ist erfindungsgemäß jeweils ein Drehgriff zur linearen Verstellung von Hand vorgesehen; jeder der Drehgriffe kann durch einen elektrischen, pneumatischen oder hydraulischen Antrieb ersetzt werden.

Außerdem hat es sich als günstig erwiesen, den Arbeitskopf durch einen Gelenkstift an eine Tragzunge eines Zwischenstückes der Führungsvorrichtung drehbar anzuschließen, welches andernends verschieblich an einem mit dem Festpunkt verbundenen Schlitten lagert. Dank des Schlittens ist das Zwischenstück mit dem Arbeitskopf parallel zur Längsachse der Führungsvorrichtung reversierbar.

In Abstand zu jenem Gelenkstift kann die Tragzunge am Zwischenstück um die Längsachse -- also rechtwinkelig zur Achse des Gelenkstiftes -- drehbar befestigt sein. Dazu ragt die Tragzunge von einer Drehscheibe ab, die mit einer Welle verbunden ist und drehbar einer scheibenartigen Lagerfläche des Zwischenstücks anliegt. Die bevorzugt in einem Winkel von 360° drehbare Drehscheibe ist über jene Welle mit einem Stellknopf verbunden, durch den sie in gewünschter Drehstellung fixiert zu werden vermag.

Sowohl der quer zur Längsachse zu verschiebende als auch anhebbare Frontschlitten am Arbeitskopf als auch der erfindungsgemäß über eine Verstellspindel mit dem Zwischenstück verbundene Heckschlitten, dessen Bewegungsbahn parallel zur Längsachse der Führungsvorrichtung verläuft, sind jeweils mit einem Drehgriff zu ihrer Betätigung versehen. Will man von Handbetrieb auf automatischen Betrieb umstellen, werden diese Drehgriffe erfindungsgemäß selbst als Motoren oder Antriebe ausgebildet oder durch solche ersetzt.

So liegt es im Rahmen der Erfindung, daß der Arbeitskopf in mehreren Achsen durch die -- Feingewinde aufweisenden --Schlitten mit elektrischen, elektronischen, pneumatischen oder hydraulischen Antrieben verstellbar ausgebildet ist. Dabei werden zum einen Elektro-Schritt-für-Schritt-Motoren oder elektronische Magnetmotoren als Antriebe bevorzugt. Zum anderen sollen pneumatische Antriebe vor allem mit komprimierter Luft oder komprimiertem Stickstoff als Antriebsmedium betrieben werden; für hydraulische Antriebe werden Wasser oder Öl als Antriebsmedium bevorzugt; diese sollen nach einem weiteren Merkmal der Erfindung in einem geschlossenen Kreislauf geführt werden.

Erfindungsgemäß wird zur exakten Kontrolle der Bewegungen dem/den Betätigungsorgan/en oder Drehgriff/en für den/die Schlitten und/oder die Drehachse bevorzugt zumindest eine Anzeigeeinrichtung für die Einstelldaten zugeordnet.

Nach weiteren Maßgaben des Erfinders können an dem/den Schlitten oder der/den Drehachse/n Meßskalen vorgesehen werden, denen gegebenenfalls zumindest ein elektromechanisches Ablesesystem zugeordnet ist.

Von Bedeutung für die Erfindung kann auch sein, daß die gesamte beschriebene Anlage über einen Computer eingestellt und nachgesteuert werden kann, bevorzugt über eine in den Computer eingegebene Röntgenaufnahme; dank letzterer kann man die Anlage sogar automatisch einstellen und nachsteuern.

Setzt man voraus, daR auf die Bewegungseinheit bzw. den in mehreren Achsen gesteuerten Arbeitskopf jedes -- bevorzugt in der Chirurgie oder Kieferchirurgie einsetzbare -- Bohr- oder Fräsgerät aufgebracht werden kann, ergibt sich ein System, das die vom Erfinder gesehene Aufgabe in bestechender Weise löst.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Fig. 1:: eine Schrägsicht auf eine Führungsvorrichtung mit Arbeitskopf zur Aufnahme eines Instruments;
- Fig. 2:: eine vergrößerte Schrägsicht auf den verstellbare Achsen aufweisenden Arbeitskopf;
- Fig. 3:: eine Schrägsicht auf eine Verschiebeeinrichtung für den Arbeitskopf;

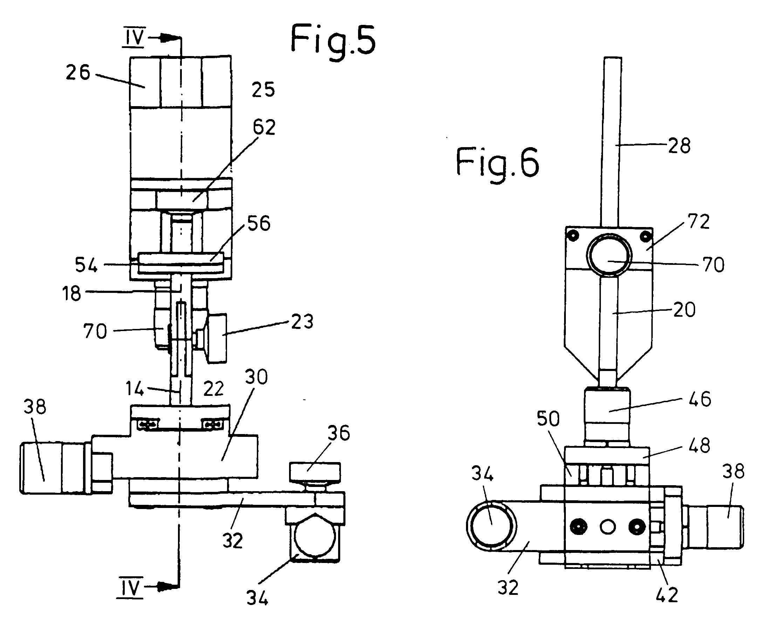

- Fig. 4:: den Längsschnitt durch die Führungsvorrichtung nach Linie IV-IV in Fig. 5;
- Fig. 5:: die Ansicht der Führungsvorrichtung von unten;
- Fig. 6:: die Frontansicht der Führungsvorrichtung.

Eine Führungsvorrichtung 10 für Werkzeuge, vor allem für medizinische Instrumente, weist an ihrer Basis einen Arbeitskopf 12 mit laschenartig abragendem Kupplungsarm 14 auf; letzterer lagert in einem Aufnahmeschlitz 16 einer gabelartig ausgebildeten Tragzunge 18 eines Zwischenstückes 20 und ist mit der Tragzunge 18 durch einen Gelenkstift 22 drehbar verbunden; letzterem ist an einem freien Ende ein Handknopf zum Auslösen einer Drehbewegung in Pfeilrichtung a zugeordnet.

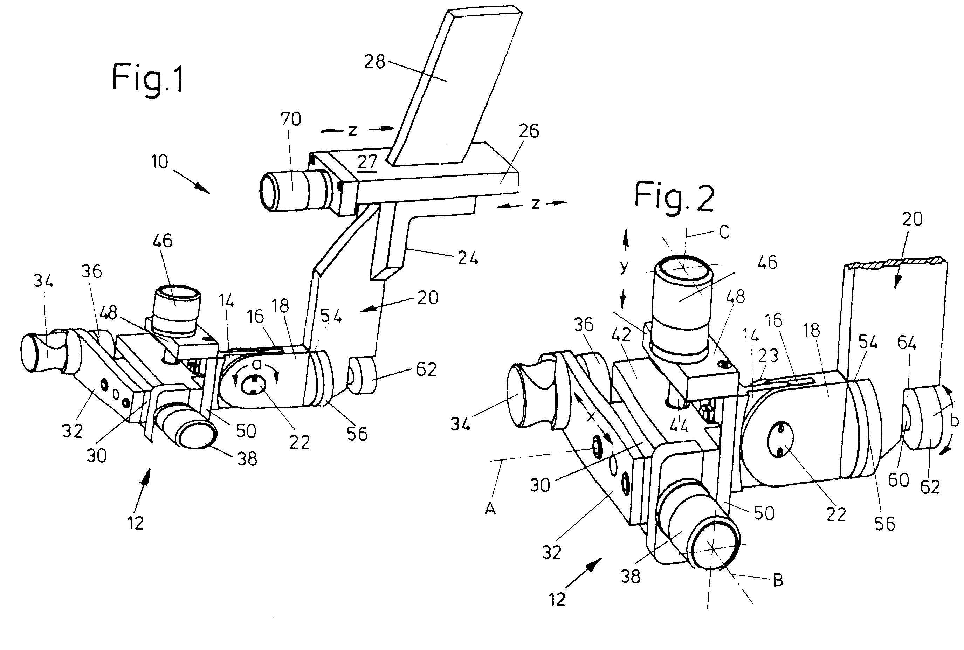

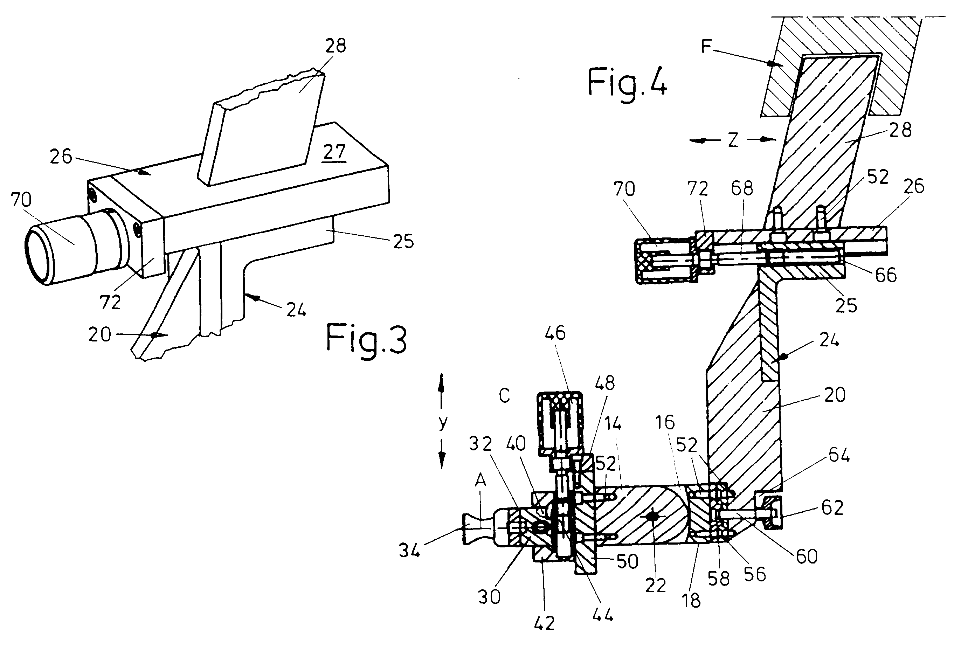

An dem in Fig. 1 oberen Ende des plattenartigen Zwischenstückes 20 lagert auf einem Winkelsockel 24 ein flacher Heckschlitten 26, von dessen Oberfläche 27 ein Werkstoffstreifen 28 flossenartig aufragt. Mittels dieses als Halteteil dienenden Werkstoffstreifens 28 kann die Führungsvorrichtung 10 an einem -- in der Zeichnung aus Gründen der Übersichtlichkeit lediglich in Fig. 4 bei F angedeuteten -- Festpunkt, beispielsweise an einem stabilen Ständer, befestigt werden.

Am Arbeitskopf 12 ist ein -- quer zur Längsachse A der Führungsvorrichtung 10 bewegbarer -- Frontschlitten 30 vorgesehen, an dem eine ihn einseitig überragende Lasche 32 festliegt; von dieser kragt ein zu jener Längsachse A paralleler Instrumentenhalter oder Befestigungsring 34 für einen nicht dargestellten Bohr- oder Fräskopf ab. Dieser Befestigungsring 34 ist jenseits der Lasche 32 mit einem Feststellknopf 36 versehen.

Parallel zur Lasche 32 verläuft die Achse B eines seitlich abragenden Drehgriffes 38, mit dem eine horizontale Bewegung -- Pfeil x -- des Frontschlittens 30 für den Befestigungsring 34 ausgelöst zu werden vermag. Vor allem Fig. 4 verdeutlicht, daß dazu der Frontschlitten 30 in einer Bahn geführt wird, die von einer Ausnehmung 40 einer zur Lasche 32 parallelen, in Fig. 4 horizontalen Schiene 42 angeboten wird. Letztere ist mit einem zu ihr lotrechten Gestänge 44 eines weiteren Drehgriffs 46 verbunden, dank dessen die Schiene 42 -- und mit ihr der Frontschlitten 30 -- in Pfeilrichtung y heb- und senkbar ist; die Achse dieses Gestänges 44 ist mit C bezeichnet.

Der Drehgriff 46 ist auf einem Werkstoffstreifen 48 vorgesehen, der seinerseits an einer -- quer zur Längsachse A kopfseitig den Kupplungsarm 14 vorgesetzten und an ihn angeschraubten -- vertikalen Platte 50 sitzt. Die den Kupplungsarm 14 aufnehmende Tragzunge 18 ist andernends durch Schrauben 52 mit einer Drehscheibe 54 fest verbunden, die einer entsprechend kreisförmigen Lagerfläche einer Lagerscheibe 56 an der Basiskante 58 des plattenförmigen Zwischenstücks 20 anliegt.

Die an eine etwa in der Längsachse A verlaufende Welle 60 angefügte Drehscheibe 54 ist -- in Pfeilrichtung b -- in einem Winkel von 360° um die Längsachse A drehbar und in ihrer jeweils gewählten Lage mittels eines Stellknopfes 62 festzulegen; dieser befindet sich in einer Eckausnehmung 64 des Zwischenstücks 20.

Wie bereits erwähnt, trägt das Zwischenstück 20 an seinem oberen Ende den Winkelsockel 24, dessen einer -- querschnittlich parallel zur Längsachse A stehender -- Schenkel 25 in einem Kanal 66 eine Verstellspindel 68 aufnimmt. Diese ist mit ihrem freien Ende an einen Drehgriff 70 angeschlossen und bewirkt bei dessen Betätigung eine Bewegung des Heckschlittens 26 in Pfeilrichtung z. Ein von der Verstellspindel 68 durchsetzter Frontstreifen 72 des Heckschlittens 26 dient dem Drehknopf oder Drehgriff 70 als Widerlager.

Da -- wie erwähnt -- der mit dem Heckschlitten 26 fest verbundene flossenartige Werkstoffstreifen 28 als Halteflosse anderseits an einen Festpunkt F angeschlossen ist, bewirkt der Heckschlitten 26 bzw. die Betätigung von dessen Drehknopf 70 die Vorwärts- und Rückwärtsbewegung -- Pfeil z --des in den Achsen A, B, C verschieblichen und um den Gelenkstift 22 bzw. die Welle 60 drehbaren Arbeitskopfes 12.

Die beschriebene Führungsvorrichtung 10 dient nach dem Anbringen eines -- handelsüblichen -- Bohr- oder Fräskopfes am Arbeitskopf 12 der Herstellung von Vertiefungen und Bohrungen in einem Knochengewebe, um Implantate mit hoher Präzision einsetzen zu können. Der Arbeitskopf 12 kann in mehreren Achsen durch die Feingewinde aufweisenden Schlitten 26, 30, 42 bzw. die Drehgelenke 22, 54/56 von Hand oder aber elektrisch, elektronisch, pneumatisch oder hydraulisch verstellt werden; durch Austausch der beschriebenen Stellknöpfe oder Drehgriffe 38, 46, 70, die es erlauben, jene Schlitten 30, 42, 26 zu bewegen, gegen jeweils einen elektrischen, elektronischen, magnetischen, pneumatischen oder hydraulischen Motor bzw. Antrieb besteht die Möglichkeit einer Automatisation, die es zudem gestattet, die Führungsvorrichtung 10 über einen Computer anzusteuern und auf diese Weise die Bohrungen, Langlöcher etc. in der gewünschten Präzision herzustellen.

Die entsprechenden Antriebe sind in der Zeichnung ebenso vernachlässigt wie zugehörige Stromkabel oder Versorgungsleitungen für diesbezügliche Strömungsmittel. Nicht erkennbar sind zudem Sensoren und Meßskalen, mit denen die exakten Meßdaten eingestellt bzw. aufgenommen werden können. Denn das Verstellen oder Bewegen der einzelnen Achsen A, B, C; 22, 54/56 von Hand oder über eine Antriebseinheit kann während des Arbeitsvorganges über -- an den einzelnen Bewegungsbereichen 26, 30, 42 bzw. 22, 54/56 angeordnete und nicht dargestellte -- Meßskalen, an denen durch Meßsensoren auf einem Bildschirm oder eine Meßdatenanzeigeeinrichtung übertragene Werte während der Operation erscheinen, kontrolliert und im Notfall korrigiert werden.

Bei einer computergesteuerten Führungsvorrichtung 10 ist das Bewegungsprogramm für den Eingriff durch Bohren und/oder Fräsen vorher eingebbar, so daß die eigentliche Bohr- und/oder Fräsbewegung vollautomatisch durchgeführt zu werden vermag.

## Patentansprüche

1. Führungsvorrichtung für Instrumente, insbesondere für chirurgische Instrumente zum Herstellen von Kavernen wie Vertiefungen und Bohrungen für Implantate in Knochengeweben,
dadurch gekennzeichnet,
daß ein das Instrument aufnehmender Instrumentenhalter (34) an der an einem Festpunkt (F) festlegbaren Führungsvorrichtung (10) in wenigstens zwei einander querenden Richtung (x, y, z) verlagerbar sowie um zumindest eine Gelenkachse (A) drehbar angeordnet ist.

2. Führungsvorrichtung nach Anspruch 1, dadurch gekennzeichnet, daß der Instrumentenhalter (34) in drei Koordinatenrichtungen (x, y, z) verschiebbar gelagert ist.

3. Führungsvorrichtung nach Anspruch 1 oder 2, dadurch gekennzeichnet, daß der Instrumentenhalter (34) um die Gelenkachse (A) in einem Winkel von mehr als 90°, bevorzugt 360°, drehbar gelagert ist.

4. Führungsvorrichtung nach einem der Ansprüche 1 bis 3, dadurch gekennzeichnet, daß der Instrumentenhalter (34) um eine die Gelenkachse (A) querende zweite Gelenkachse (22) schwenkbar angeordnet ist.

5. Führungsvorrichtung nach einem der Ansprüche 1 bis 4, dadurch gekennzeichnet, daß der an einem Arbeitskopf (12) der Führungsvorrichtung (10) angebrachte Instrumentenhalter (34) quer zur Längsachse (A) der Führungsvorrichtung an einem Schlitten (30) verschiebbar sowie mit diesem anhebbar ausgestaltet ist, wobei gegebenenfalls der Instrumentenhalter (34) seitlich des Arbeitskopfes (12) an einer diesen überragenden Lasche (32) od.dgl. Streifen angebracht und/oder wobei für die die Längsachse (A) querende Bewegungsbahn des Instrumentenhalters (34) sowie die Hubbahn des Arbeitskopfes (12) jeweils ein Drehgriff (38, 46) vorgesehen ist.

6. Führungsvorrichtung nach einem der Ansprüche 1 bis 5, dadurch gekennzeichnet, daß der Arbeitskopf (12) durch einen Gelenkstift (22) an eine Tragzunge (18) eines Zwischenstückes (20) angelenkt ist, wobei gegebenenfalls die Tragzunge um die Längsachse (A) drehbar am Zwischenstück angeordnet ist.

7. Führungsvorrichtung nach einem der Ansprüche 3 bis 6, dadurch gekennzeichnet, daß die Tragzunge (18) von einer Drehscheibe (54) abragt, die an einer Welle (60) festgelegt und drehbar einer Lagerfläche (56) des Zwischenstücks (20) zugeordnet ist, wobei gegebenenfalls die Drehscheibe durch die Welle mit einem Stellknopf (62) verbunden ist.

8. Führungsvorrichtung nach Anspruch 6 oder 7, dadurch gekennzeichnet, daß das Zwischenstück (20) an einem Schlitten (26) verfahrbar lagert, der anderseits mit dem Festpunkt (F) verbunden ist, wobei gegebenenfalls der Schlitten des Zwischenstücks parallel zur Längsachse (A) der Führungsvorrichtung (10) bewegbar vorgesehen ist und/oder wobei die Bewegungsbahn des Schlittens (26) parallel zur Längsachse (A) der Führungsvorrichtung (10) verläuft.

9. Führungsvorrichtung nach Anspruch 8, dadurch gekennzeichnet, daß der Schlitten (26) über eine Verstellspindel (68) mit dem Zwischenstück (20) verbunden und die Verstellspindel mit einem Drehgriff (70) zu ihrer Betätigung versehen ist.

10. Führungsvorrichtung nach wenigstens einem der Ansprüche 1 bis 9, dadurch gekennzeichnet, daß dem/den Betätigungsorgan/en oder Drehgriff/en (23, 38, 46, 70) für den die Schlitten (26, 30, 42) und/oder die Drehachse/n (22, 66) zumindest eine Anzeigeeinrichtung für die Einstelldaten zugeordnet ist, wobei gegebenenfalls an dem/den Schlitten (26, 30, 42) oder der/den Drehachse/n (22, 66) Meßskalen angeordnet sind.

11. Führungsvorrichtung nach Anspruch 10, gekennzeichnet durch ein der/den Meßskala/skalen zugeordnetes elektromechanisches Ablesesystem oder durch über eine Direktablesung ablesbar ausgebildete Wegsensoren der Meßskalen.

12. Führungsvorrichtung nach Anspruch 10, dadurch gekennzeichnet, daß den Meßskalen an dem/den Schlitten (26, 30, 42) oder der/den Drehachse/n (22, 66) Wegsensoren zugeordnet und diese mit einem Rechner verbunden sind.

13. Führungsvorrichtung nach Anspruch 5 oder 9, dadurch gekennzeichnet, daß der Drehgriff (38, 46, 70) als Antrieb oder Motor ausgebildet ist.

14. Führungsvorrichtung nach Anspruch 13, dadurch gekennzeichnet, daß der Arbeitskopf (12) in mehreren Achsen (A, B, C) durch die Feingewinde aufweisenden Schlitten (26, 30, 42) mit elektrischen, elektronischen, pneumatischen oder hydraulischen Antrieben verstellbar ausgebildet ist und/oder daß dem/den Schlitten (26, 30, 42) ein Elektro-Schritt-für-Schritt-Motor als Antrieb zugeordnet oder ein elektronischer Magnetmotor als Antrieb zugeordnet ist.

15. Führungsvorrichtung nach Anspruch 13 oder 14, gekennzeichnet durch komprimierte Luft oder durch komprimierten Stickstoff als Antriebsmedium.

16. Führungsvorrichtung nach Anspruch 13 oder 14, gekennzeichnet durch Wasser oder durch Öl als Antriebsmedium, wobei gegebenenfalls das Wasser oder das Öl als Antriebsmedium in einem geschlossenen Kreislauf geführt ist.
